# EUROPEAN PATENT APPLICATION

(11) **EP 1 180 380 A2**
(43) Date of publication of application: **20.02.2002**
(21) Application number: 01119554.2
(22) Date of filing: 14.08.2001
(51) Int. Cl.: A61M 25/01, A61B 18/00

(54) **Catheter apparatus for medical treatment**

(30) Priority: 17.08.2000 US 225824 P
(71) Applicant: Borkan, William N., North Miami Beach, Florida 33160 (US)
(72) Inventor: Borkan, William N., North Miami Beach, Florida 33160 (US)
(74) Representative: Strehl Schübel-Hopf & Partner

(57) **Abstract**

A method including administering a drug to the patient and positioning a catheter adjacent to the tissue to be treated by the drug. The tissue may then be stimulated using the catheter. The catheter may include one or more contacts or optical parts at the distal end if the catheter stimulation is by electric energy or by photonic energy, respectively. If the catheter includes a passage with one or more ports at the distal end of the catheter, the drug is administered through the passage. Alternatively, drugs may be administered systematically or concurrently with intrathecally administered agents.

## Description

### CROSS-REFERENCE

This application claims priority of Provisional Application serial No. 60/225,824 filed August 17, 2000.

### BACKGROUND AND SUMMARY OF THE INVENTION

The present invention relates to implanted and temporarily implanted catheters and their use in medical treatments or procedures.

The concept of using catheters for various medical procedures is well known.

The catheter of the present invention is designed in one embodiment for use as part of a method of repairing a bone or cartilage percutaneously. This method includes positioning the catheter adjacent to bone or cartilage to be repaired and dispensing a fluid through the catheter. The fluid is then activated using the catheter. The fluid may be an adhesive. The catheter includes one or more electrodes at the distal end and the activation is by electric energy. Wherein the catheter includes an optical channel with one or more ports at the distal end, the activation is by photonic energy. Wherein the fluid is responsive to light energy of a specific wavelength, activation of the fluid is with light energy of that specific wavelength. Wherein the fluid is responsive to heat or cold, activation of the fluid is with heat or cold using in the catheter. The heat may be produced by electric or photonic energy. The catheter can include an additional optical channel with a port at the distal end in the catheter to position using the optical channel.

Another application of the instant invention is administration of healing or growth enhancing substances and/or treatments. For instance, the use of electrical stimulation to enhance bone growth is well known. There are also certain drugs and chemicals which are known to hasten healing and repair of fractured bones. This invention provides a unique and novel method of positioning a catheter (with optional camera assisted placement) of a catheter adjacent to tissues (bone) to be treated and administering a combination of stimulation and drug therapy.

It is a further object of the invention that this treatment may either be limited in duration such that the catheter is inserted percutaneously for a short period of time; of medium duration with an implanted battery and limited quantity of drug or chemical; or long term utilizing internally or externally powered stimulation circuitry, a drug pump, reservoir and port.

Advances in catheter technology have allowed the widespread delivery of drugs for various medical applications. In addition, various fixation means for catheters have been developed and successfully utilized to eliminate the problem of electrode movement and migration. Therefore, it is now possible to develop a catheter electrode for placement into the human body either temporarily or permanently without the problems and complications experienced previously.

The present invention provides various leads or catheters to lie along and treat tissue. The electrodes on the leads may be various sizes to conserve the battery as well as allow a more defined area of stimulation. It may also include multiple channels or passages for delivery of drugs; thermal or photonic energy.

The sheath may include a fixing element configured to fix the catheter in place along the tissue. The fixing element may include at least one of the following: inflatable balloons, nitinol, tines and the sheath shape itself.

The sheath may also include a passage extending from an inlet at the proximal end of the sheath to one or more outlets at the distal end of the sheath. The outlets may be located at one or more of the tip of the distal end, the area between the electrodes and on the electrodes. This passage may be used for dispensing of drugs or other fluids, for example, adhesive. It may also be an optical channel or for a stilet to be used during positioning of the lead. This may be used without fixing elements.

Alternatively, one or more optical channels can be provided extending from a port at the proximal end of the sheath to a port at the distal end of the sheath. The port for the optical channel at the distal end may be located at one or more locations including, but not limited to, the tip of the distal end, the area between the electrodes and on the electrodes. The optical channel can provide photonic energy to the tissue as well as functioning as a lens for a remote camera to assist in proper positioning of the catheter and to monitor dispensing of the drug or adhesive.

The catheter of the present invention can also be used for a drug treatment method. A method including administering a drug to the patient and positioning a catheter adjacent to the tissue to be treated by the drug. The tissue may then be stimulated using the catheter. The catheter may include one or more contacts or optical parts at the distal end if the catheter stimulation is by electric energy or by photonic energy. Respectively, if the catheter includes a passage with one or more ports at the distal end of the catheter, the drug is administered through the passage. Alternatively, drugs may be administered systematically or concurrently with intrathecally administered agents.

The drug administered may be selected to be responsive to stimulation. Wherein the drug is responsive to light energy with a specific wavelength, the stimulation of the tissue is with the light energy of that specific wavelength using the catheter. Wherein the drug is responsive to heat, the tissue is stimulated with heat using the catheter. The heat may be produced by electrical energy or photonic energy. The cold may be produced by a Peltier effect device or other means for example gas or liquids. An additional optical channel with a port at the distal end of the catheter may be provided and is used to position the optical channel.

It is a further object of this invention to allow various chemical and biochemical substances to be administered into the human body which may be simultaneously activated by a form of energy using the same device (catheter) for administration and activation.

Other objects, advantages and novel features of the present invention will become apparent from the following detailed description of the invention when considered in conjunction with the accompanying drawings.

### BRIEF DESCRIPTION OF THE DRAWINGS

Figure 1 is a perspective view of a catheter lead including a passage having an outlet at the tip of the electrode and a balloon fixation device. Provision for an optional stilet is also shown.

Figure 2 is a perspective view of a catheter lead with outlets between the electrodes and a nitinol fixation device deployed according to the principles of the present invention.

Figure 2A shows a cutaway view of the nitinol fixation device in position prior to deployment.

Figure 3 is a perspective view of a catheter lead with a passage having an outlet on the electrode and a tine fixation device according to the principles of the present invention.

Figure 4 is a perspective view of a catheter lead wherein the passage is external with the sheath according to the principles of the present invention.

Figure 5 is a perspective view of a catheter electrode with a passage as well as two optical channels according to the principles of the present invention.

Figure 6 is a perspective view of a catheter lead with an additional common anode electrode.

Figure 7 illustrates a bone or cartilage repair according to the principles of the present invention.

### DETAILED DESCRIPTION OF THE PREFERRED EMBODIMENTS

The present invention describes a catheter capable of delivering a fluid and stimulating the delivered fluid, for example, drugs or which can be used to repair tissue or cartilage.

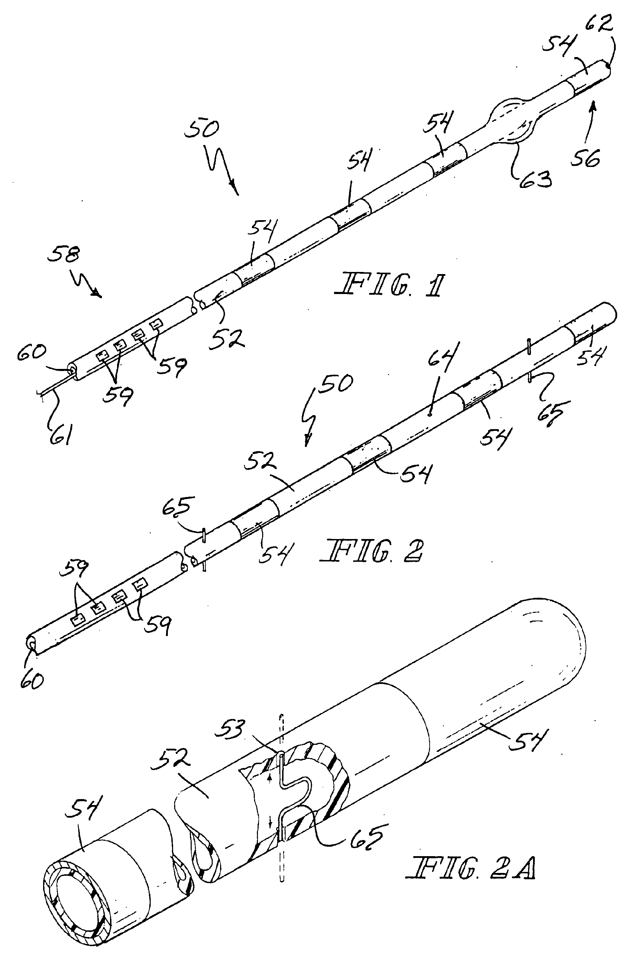

A catheter lead capable of stimulation electrically as well as dispensing fluids is illustrated in Figures 1-6. The catheter lead 50 has a sheath 52 with inline electrode 54 spaced along at the distal end 56. At the proximal end 58, terminal contacts 59 are connected internally to each of the electrodes shown. A passage 60 is provided in the sheath 50. In Figure 1, an outlet 62 is provided in the tip of the distal end 56 and a balloon fixation device 63 is shown. Provision is also made for an optional stilet which is removable and may be used to assist in placement of the catheter. Various stilets 61 of different shapes and characteristics may be used with these leads.

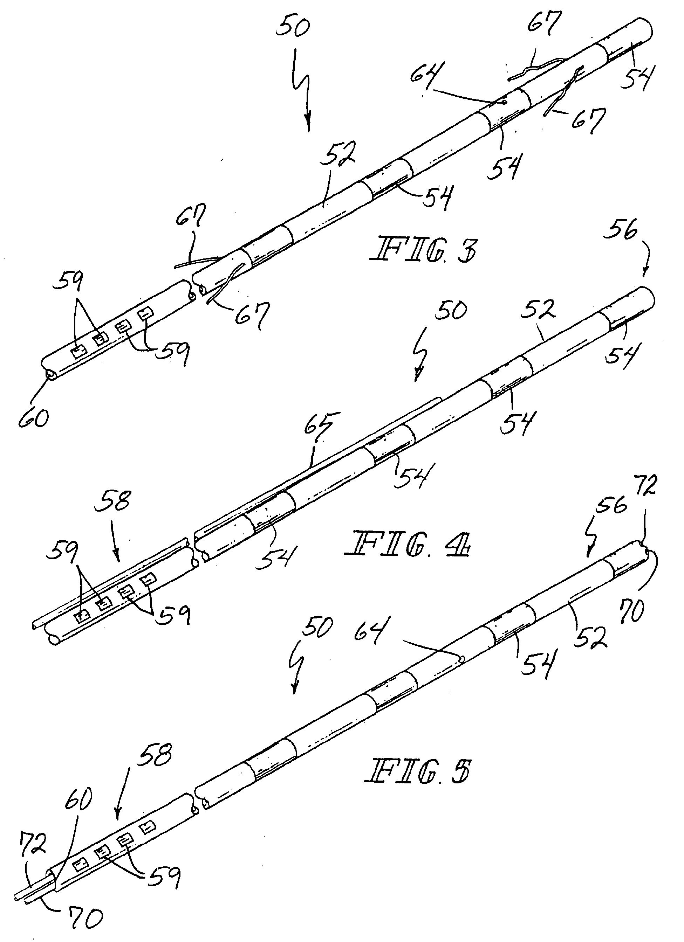

In Figure 2, the outlet 64 is shown in the space between the electrodes 54 and a nitinol fixation device 65 is shown in the deployed condition. In figure 2A, nitinol fixation device 65 is shown prior to the deployment through opening 53 in sheath 52. In Figure 3, the outlet 64 is shown in or on the electrode 54 and a tine fixation device 67 is shown.

Although one fixation is illustrated on a specific Figure, any fixation device may be used with any of the catheters. Fixation methods may also include devices that are actively deployed and/or retracted (for instance by a stilet) in addition to the methods shown herein. Also, the fixation device may be located at any or more than one location or position along the catheter. A fixation device should be used where the catheter electrode is installed in the intrathecal space. In other uses, the catheters may be used without fixation devices.

While Figures 1-3 show the passage 60 internal to the sheath 52, an external delivery 68 may be used as shown in Figure 4.

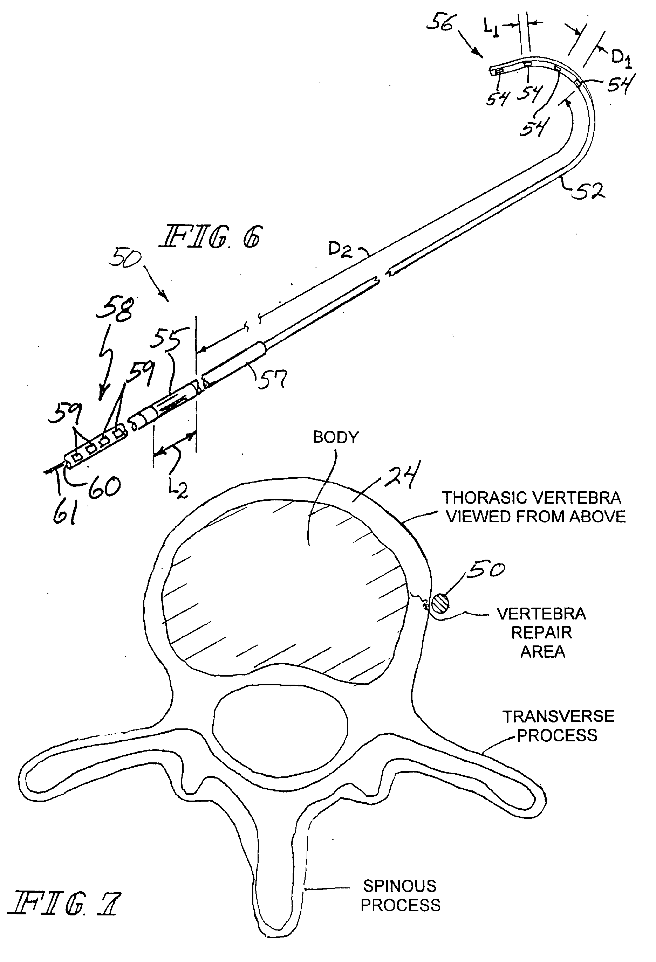

The electrode catheter 50 may also include a single or pair of optical channels 70 and 72 having outlets or ports in the distal end as illustrated in Figure 5. The fiberoptic channels and light energy delivered through a clear translucent area in the catheter. One of the channels can provide a source of light to be used as a further source of stimulation. The other channel will form a lens for a camera or other monitoring devices. The camera can be used in positioning the electrode or distal end. Channel 60 with outlet 64 is also shown for a drug delivery.

It should be noted that only a single optical path can be used to provide a source of light for a photon stimulation without the passage 60 or the outlet 64. It should also be noted that the passage 60 or external passage 68 for drug or other fluid delivery may be used in combination with the light channel 70 without the electrodes 54. Although two channels 70, 72 are shown, any number of channels could be used and could include a combination of different types of channels - working channels for instruments , optical channels for light or camera, stilets etc.

The lead 50, as illustrated in Figure 6, is curved at its distal end 56 which includes the electrodes 54. Sheath 52 includes a wire extension 57 which includes the additional anode electrode 55 and the electrical contact 59. The curved distal end 56 wraps around the spinal cord or the nerves. This is another form of a fixing device. The stilet 61 can be inserted through passage 60 to maintain the distal end 56 linear until it is adjacent to the nerve or the spinal cord. The passage 60 may then be used for fluid delivery.

The electrode 54 all have a length L1 and the additional electrode 55 has a length L2. Length L2 is greater than L1, at least twice its length. Thus, for example, if lead L1 is two millimeters, the length L2 is four millimeters. The length L2 may be anywhere between 2-4 times the area of the length L1. Also, it should be noted that the additional electrode 55 is spaced by a distance D2 from the nearest electrode 54. Where D1 is approximately six millimeters, the distance D2 is at least 10 millimeters and can be as much as 20 millimeters. With this distance, the electrodes 54 act as a point source when used in conjunction with the additional electrode 55 of the increased area. The electrode 54 may be cathodes with electrode 55, a common anode. The difference in surface area of the additional electrode 55 compared to the other electrodes 54 may be by their difference in circumferential dimension.

As illustrated in the Figure 6, the electrodes 54 may have 180° circumferential dimension or less compared to a 360° of the additional electrode 55. Thus, their lengths could be the same to achieve the difference in surface area.

For bone or cartilage repair, the catheter 50, for example, that of Figure 5, is positioned adjacent a repair area 22 a bone or cartilage 24 as shown in Figure 7. This may use the aid of a camera or scope using light channel 72. A fluid, for example, an adhesive is dispensed through passage 60 of the catheter to the bone or cartilage. The adhesive is then activated using the electrode 54 or photonic energy from light channel 70. The adhesive is selected such that it is activated upon stimulation. This may be by heat, cold, light or electrical energy. Surgical adhesives that are activated by a specific wavelength of light are well known and used, for example, in dentistry. This method is a percutaneous method using the catheter. The bone or cartilage may be in the vertebrae or in any part of the skeleton. This allows percutaneous repair without suturing or major incisions. The fluid may also be a drug or chemical which promotes healing or growth of bone cartilage or tissue with stimulation.

The catheter of Figures 1-6 may also be used in a percutaneous drug treatment method. The drug is administered to the patient and the catheter is positioned adjacent the tissue to be treated by the drug. The tissue is then treated using the catheter. This allows selective and localized drug treatment with or without stimulation. Certain compounds change chemically when stimulated. Compounds can also be delivered via electropheretic means.

The source of stimulation and/or the drugs may be external to the body or totally implantable. The implantable system could include a microprocessor , pump, port and an external port for refilling the pump or selection of a different drug or fluid.

The drug may be stimulated by electrical energy using the electrodes 54 or by photonic energy using the optic channel 70. Heat may be produced by either the electrodes 54 or the optical channel 70. Cold may be produced by a Peltier effect chip or other means, for example gas or liquids. If the drug is responsive to light energy of a specific wavelength, the stimulation uses light energy of that specific wavelength. It should be noted that the application of light or photonic energy to stimulate tissue with or without drug treatment may be used in any part of the body, for example, the eye, optic nerve, auditory nerve, brain, etc.

Although the present invention has been described and illustrated in detail, it is to be clearly understood that the same is by way of illustration and example only, and is not to be taken by way of limitation. The spirit and scope of the present invention are to be limited only by the terms of the appended claims.

## Claims

1. A method of drug treatment comprising:
administering a drug to a patient;
positioning a stimulation catheter adjacent tissue to be treated by the drug; and
stimulating the tissue using the catheter.

2. An apparatus for drug treatment comprising:
means for administering a drug to a patient;
a stimulation catheter adapted for positioning adjacent tissue to be treated by the drug; and
means for stimulating the tissue using the catheter.

3. The invention of claim 1 or 2, wherein the catheter includes one or more electrodes at the distal end and the stimulation is by electric energy.

4. The invention of claim 1 or 2, wherein the catheter includes an optical channel with one or more ports at a distal end of the catheter and the stimulation is by photonic energy.

5. The invention of claim 1 or 2, wherein the catheter includes a passage with one or more ports at a distal end of the catheter and the drug is administered through the passage.

6. The invention of claim 1 or 2, wherein the drug is administered systemically.

7. The invention of claim 1 or 2, wherein the drug administered is selected to be responsive to stimulation.

8. The invention of claim 7, wherein the drug administered is responsive to light energy of a specific wave length and including stimulating the tissue with light energy of the specific wave length using the catheter.

9. The invention of claim 7, wherein the drug administered is responsive to heat and including stimulating the tissue with heat using the catheter.

10. The invention of claim 7, wherein the drug administered is responsive to cold and including stimulating the tissue with cold using the catheter.

11. The invention of claim 3, wherein the catheter includes an optical channel with a port at a distal end of the catheter and the catheter is positioned using the optical channel.

12. A method of repairing a bone or cartilage percutaneously comprising:
positioning a catheter adjacent bone or cartilage to be repaired;
dispensing a fluid through the catheter; and
activating a fluid using the catheter.

13. An apparatus of repairing a bone or cartilage percutaneously comprising:
a catheter adapted for positioning adjacent bone or cartilage to be repaired;
means for dispensing a fluid through the catheter; and
means for activating the fluid using the catheter.

14. The invention of claim 12 or 13, wherein the catheter includes one or more electrodes at a distal end and the activation is by electric energy.

15. The invention of claim 12 or 13, wherein the catheter includes an optical channel with one or more ports at a distal end of the catheter and the activation is by photonic energy.

16. The invention of claim 12 or 13, wherein the fluid is responsive to light energy of a specific wave length and including activating the fluid with light energy of the specific wave length using the catheter.

17. The invention of claim 12 or 13, wherein the fluid is responsive to heat and including activating the fluid with beat using the catheter.

18. The invention of claim 12 or 13, wherein the fluid is responsive to cold and including activating the fluid with cold using the catheter.

19. The invention of claim 12 or 13, wherein the catheter includes an optical channel with a port at a distal end of the catheter and the catheter is positioned using the optical channel.

20. The invention of claim 12 or 13, wherein the fluid is an adhesive which adheres to the bone or cartilage.

21. The invention of claim 12 or 13, wherein the fluid enhances bone, cartilage or tissue growth.
